# EUROPEAN PATENT SPECIFICATION

(11) **EP 4 412 569 B1**
(45) Date of publication and mention of the grant of the patent: **07.01.2026**
(21) Application number: 21794480.0
(22) Date of filing: 08.10.2021
(51) Int. Cl.: A61F 13/511, A61F 13/512, A61F 13/15

(54) **ABSORBENT ARTICLE**
ABSORBIERENDER ARTIKEL
ARTICLE ABSORBANT

(43) Date of publication of application: 14.08.2024
(73) Proprietor: Essity Hygiene and Health Aktiebolag, 405 03 Göteborg (SE)
(72) Inventor: BLOMSTRÖM, Philip, 405 03 Göteborg (SE); PALMQVIST, Lisa, 405 03 Göteborg (SE); KNÖS, Anna, 405 03 Göteborg (SE)
(74) Representative: Hoffmann Eitle
(86) International application number: PCT/EP2021/077900
(87) International publication number: WO 2023/057075

(56) References cited:
- EP-A1- 0 983 758
- EP-B1- 2 938 310
- WO-A1-2020/004648
- WO-A1-2021/168513
- WO-A1-97/00057
- GB-A- 2 262 906
- US-A- 6 093 871
- US-A1- 2011 224 639
- US-B2- 10 888 469

## Description

### TECHNICAL FIELD

The disclosure relates to a washable and reusable absorbent undergarment as well as a washable and reusable absorbent assembly.

### BACKGROUND

An absorbent article is worn for the purpose of absorbing body fluids such as urine and vaginal fluids. A washable and reusable absorbent undergarment comprises fabrics of woven or knitted materials. Conventionally such an absorbent undergarment comprises an integral absorbent assembly, pad or gusset located in the crotch region of the wearer when the garment is worn. After use the absorbent garment is washed or laundered before reuse. The reusable absorbent articles have been mainly supplied for low and moderate volumes of body fluids. For sustainable reasons there has been an increased demand from users for reusable absorbent undergarments and consequently there is also a demand for such articles suitable for heavy flows of body fluids.

US 2011/0224639 A1 relates to an absorbent garment having increased surface area of absorbent material as compared to conventional absorbent garments, such as sanitary napkins, diapers, and the like. In one aspect, the absorbent garment provides increased surface area around the inner thigh area of the wearer.

Moreover, each of WO 97/00057, WO 2020/004648 A1, US 10,888,469 B2, WO 2021/168513 A1, GB 2 262 906 A, and EP 0 983 758 A1 forms part of the state of the art relative to the present disclosure.

### SUMMARY

The present disclosure is based on the insight that there is a need for a washable and reusable undergarment able to handle and absorb larger volumes of body fluids without substantial leakages.

The present invention provides a washable and reusable absorbent undergarment according to claim 1 and a washable and reusable absorbent assembly according to claim 15.

The washable and reusable absorbent undergarment has an extension in the longitudinal direction and in the transversal direction and comprises an absorbent assembly. The absorbent assembly comprises a wearer facing top layer of a knitted material, a moisture barrier and at least one absorbent layer located between the top layer and the moisture barrier. The top layer has openings therein and each opening span 2-10 stitches and there are 5-60 openings/cm².

Due to the openings in the top layer, the absorbent assembly may receive a high amount of fluid in a short time without feeling overly wet. The wells will fill up with excess fluid that would otherwise flood the surface and thus give an uncomfortable, wet feeling. The filled-up wells will be emptied continuously to the layer beneath. In case of a wicking layer directly below the top layer with openings, a high wicking and spreading capacity will improve the fluid handling even further. The fluid is distributed across the surface of the wicking layer and down through it for quick absorption and containment in the absorption layer(s), such as two absorbent layers.

The openings in the top layer each have dimensions within the range of 0.3-2.0 mm, such as 0.5-1.5 mm, such as 0.6-1.3 mm.

The top layer may have 10-50 openings/cm², such as 15-40 openings/cm², such as 15-25 openings/cm².

The openings may be through holes. The openings may be arranged in rows. The rows may be transversal rows. The openings in adjacent rows may be offset in relation to each other. A "diamond" shaped macro pattern, i.e. where holes are shifted half a step on every second row may give better directional rewet properties, more even spreading and less wetting of each row than parallel rows of holes only. If one or more hole become clogged in one direction, there is also a higher risk of leakage due to "flooding" in that direction if the holes are in parallel rows compared with a diamond shaped hole pattern.

Another feature of the diamond or offset pattern is less sensitivity towards ripping in x and y directions leading to a higher durability than an even hole pattern due to fewer holes per length unit.

The openings may be non-circular. The openings may have an elongated shape. The openings may be elongated and have a ratio of length to width of >1, such as >1.5, and such as <10, such as <5. An irregular shape of the openings, such as oval, triangular or bird's eye shaped openings may direct the flow of body liquids in different directions within the absorbent assembly.

The top layer may be of a single layer. The top layer may be a single knit fabric layer. The top layer may not be a spacer fabric. The top layer may be of a Jacquard technique. The top layer may have a basis weight of 80-200 gsm. The top layer may be of a synthetic material. The top layer may be of polyester, elastan or blends thereof.

The top layer may be a weft knit, warp knit, flat knit or circular knit. The top layer may have 2-10 wales and 2-10 courses between openings. The openings may be tuck stitches. The openings may span 2-8 stitches, such as 2-6 stitches, such as 2-3 stitches. To benefit from a fast inlet the openings should be large enough to serve as wells, together with the layer directly beneath the top layer, i.e. two or more tuck stitches in a row or in two rows after each other in the course, is beneficial.

The absorbent assembly may comprise a wicking layer. The basis weight of the wicking layer may be 180-250 gsm. The wicking layer may be of a synthetic material. The wicking layer may be of polyester, polyamid, elastan or a mixture thereof. The wicking layer may be a jersey knit. The absorbent assembly may comprise two absorbent layers. The basis weight of an absorbent layer may be 200-350 gsm. The absorbent layer may be a hydrophilic French terry material. The absorbent layer may be of a synthetic material, and may be of polyester, polyamid, elastan or a mixture thereof.

The absorbent undergarment, underwear or panty may comprise a body fabric. The fabric may define a waist opening, two leg openings and a crotch region between the leg openings. The absorbent assembly may be permanently attached to the fabric.

An absorbent assembly may be used for and as a washable and reusable absorbent pad or napkin.

### BRIEF DESCRIPTION OF THE DRAWINGS

The disclosure will be described in greater detail below with reference to the figures shown in the appended drawings, wherein
- Fig. 1: shows a top view of an absorbent undergarment with an absorbent assembly between the leg openings;
- Fig. 2: shows a close-up of a top layer with openings;
- Fig. 3: shows a front view of an absorbent undergarment;
- Fig. 4: shows a cross-sectional view of a portion of an absorbent undergarment;
- Fig. 5: shows a cross-sectional view of a portion of an absorbent undergarment including a wicking layer.

### DETAILED DESCRIPTION

Different aspects of the present disclosure will be described more fully hereinafter with reference to the enclosed drawings. The embodiments of the absorbent undergarment disclosed herein can, however be realized in many different forms, such as different sizes and absorption levels, and should not be construed as being limited to the aspects set forth herein. In all figures in the following detailed description, the same reference numerals will be used to indicate the same elements.

As used herein, the term "absorbent undergarment" refers to garments that are worn and intended to be placed against the skin of the wearer to absorb and contain body fluids such as urine and vaginal fluids including menstrual fluid. The undergarment is an underwear, underpant or panty, and not a diaper, of a fabric for female or male use. The undergarment according to the present disclosure is intended to be laundered or otherwise restored after use for reuse as a sanitary article. The absorbent assembly may be used as and for a washable and reusable absorbent pad or napkin.

By "laundering" it is meant that the absorbent undergarment may be cleaned by laundering. The absorbent undergarment may thus be subjected to an aqueous solution containing detergent without losing its structural features. This aqueous solution may be heated as part of the laundering process, e.g. to 40°C or 60°C.

The term "fabric" as used in the present disclosure may refer to single or multiple layers of fabrics. The fabric may be knitted or woven.

By "permanently attached", it is meant that the absorbent assembly is not intended to be separated from the body fabric before, during, or after use. The absorbent assembly is not necessarily directly bonded to the fabric, but instead may be attached via the leg opening seams or bonded by other means such as sealing tape. The absorbent assembly herein may or may not be permanently attached to an undergarment fabric material.

Knitting is a method of constructing a fabric by interlocking a series of loops of one or more yarns. There are two major classes of knitting namely warp and weft knitting.

Weaving is a method or process of interlacing two yarns of similar materials so that they cross each other at right angles to produce woven fabric. The warp yarns run lengthwise in the fabric and the filling threads (weft) or picks, run from side to side.

Jacquard is a system of weaving or knitting that utilizes a versatile pattern mechanism to produce intricate designs by using punch cards controlling the patterns produced. Jacquard knit may be valid for circular, flatbed, warp or weft knit.

Openings or holes created by a knitting technique is generally referred to as tuck stitches herein.

The openings may be non-circular. The openings may have an elongated shape. The openings may be elongated and have a ratio of length to width of >1, such as >1.5, and such as <10, such as <5. An irregular shape of the openings, such as oval, triangular or bird's eye shaped openings may direct the flow of body liquids in different directions within the absorbent assembly. The openings in the top layer may each have dimensions within the range of 0.3-2.0, such as 0.5-1.5 mm, such as 0.6-1.3 mm.

The dimension of the openings is measured in a conventional light microscope or stereo magnifier equipped with a ruler. The material is laid out flat and the opening dimension is measured from one side of the opening to the opposite side of the opening. The dimensions in all directions of an opening should be within the range mentioned herein, also in case of an irregular shape of the opening. In case the opening span more than 1 stitch, such as 2 stitches, i.e. more than 1 adjacent tuck stitch, the opening dimension should be measured across the opening made up of the 2 combined tuck stitches.

The top layer may be constructed of any suitable fabric, including naturally derived fibers selected from the group consisting of cotton, wool, silk, cellulose, regenerated cellulose, rayon, viscose, modal, lyocell, tencel, bamboo, hemp, flax, ramie, coir or banana. Alternatively, the top layer may be constructed of synthetic fibers selected from the group consisting of polyamide, acrylic, polyester or elastane, such as a mixture of polyester and elastane. Further, the top layer may be constructed of a blend or a mixture of naturally derived and/or synthetic fibers. The materials used for construction of the top layer should be soft and nonirritating to the skin and be readily penetrated by any body fluids. According to the present disclosure, the top layer should be launderable. The top layer may comprise between 20% and 100% naturally derived fibers, more preferably between 40% and 100% naturally derived fibers and most preferably between 60% and 100% naturally derived fibers. The top skin-facing layer comprises a water-permeable material thus allowing the body fluids to migrate to the underlying absorbent layer or layers. The top layer has openings therein, each opening span 2-10 stitches and there are 5-60 openings/cm². The top layer may have a basis weight of 80-200 gsm.

A separate wicking layer may be provided underneath the top layer. The wicking layer has wicking features to allow the moisture to spread away from the wearer and into the absorbent layer or layers. Further, wicking enables an efficient spread of the moisture therefore allowing the moisture to be received in a wider area of the underlying one or more absorbent layers. This feature is particularly relevant for users suffering from stress incontinence as spurts of urine may cause the absorbent layer to be saturated rapidly at the point of impact. By spreading the volume of the fluid over a wider receiving area in the absorbent layer, the wicking features of the wicking layer increase the overall absorptive capacity of the absorbent layer. The wicking layer may be constructed of any suitable fabric, including naturally derived fibers or mixtures thereof selected from the group consisting of cotton, wool, silk, cellulose, regenerated cellulose, rayon, viscose, modal, lyocell, tencel, bamboo, hemp, flax, ramie, coir or banana. Alternatively, the wicking layer may be constructed of synthetic fibers or mixtures thereof selected from the group consisting of polyamide, acrylic, polyester or elastane. Further, the wicking layer may be constructed of a blend or a mixture of naturally derived and/or synthetic fibers. According to the present disclosure, the wicking layer should be launderable. The wicking layer may comprise between 20% and 100% naturally derived fibers, more preferably between 40% and 100% naturally derived fibers and most preferably between 60% and 100% naturally derived fibers. The basis weight of the wicking layer may be 180-250 gsm.

The absorbent assembly for use in an absorbent undergarment may contain one or multiple absorbent layers, capable of absorbing liquid and releasing the liquid during laundering. Release of the absorbed liquid may be beneficial as it enables the absorbent undergarment to be restored for reuse. Reusable absorbent undergarments provide a more sustainable alternative to the commonly used disposable hygiene articles that are not intended to be re-used.

The absorbent layer may comprise any material capable of absorbing fluid, such as woven or nonwoven microfiber or polymer knits, fabric formed from hydrophilic fibers, absorbent fibers or powders. The absorbent layer may be of natural or synthetic fibers as described above for the other ingoing layers but may be of polyester and polyamid and containing odor treatment. The odor treatment may be silver ions, copper ions and zeolites or Polyhexamethylene biguanide, PHMB.

The absorbent layer may also comprise a material having an open cell porous structures such as high loft or synthetic fibers having reservoir properties. The absorbent layer may comprise between 20% and 100% naturally derived fibers, more preferably between 40% and 100% naturally derived fibers and most preferably between 60% and 100% naturally derived fibers. The basis weight of each absorbent layer may be 200-350 gsm. There may be one or more, such as 2, absorbent layers in the absorbent assembly.

The liquid-impermeable barrier layer may comprise any suitable material or combinations of material that prevents liquid from migrating from the absorbent assembly to the body fabric. The liquid-impermeable barrier layer may comprise a hydrophobic woven or nonwoven material having inherent hydrophobic properties, or that has been treated to become hydrophobic. Examples of hydrophobic materials for treating the barrier layer are polymers such as silicone, polyurethane and combinations thereof. The liquid-impermeable barrier layer may comprise a microporous polymer film, e.g. a polyethylene, PTFE or polyurethane film, or combinations thereof. Laminates of polymer films and nonwoven materials may also be used. The liquid-impermeable barrier layer may be a coating of a moisture-impermeable material. The coating may be a polymer such as urethane wax on the surface facing away from the wearer. The liquid-impermeable barrier layer may be of polyurethane.

The liquid-impermeable barrier layer is preferably breathable, to allow vapor to escape from the absorbent undergarment, while preventing liquid from passing through the fabric. Further, the liquid-impermeable barrier layer may be constructed of an elastic material, thus providing the absorbent assembly with the required flexibility to adapt to the user's anatomy and movements. This improved fit increases the wearer's comfort during use and helps to prevent leakage from migrating through the undergarment's leg openings.

The absorbent undergarment, underwear, underpants or panty comprises a body fabric. The body fabric layer may define a waist opening, two leg openings and a crotch region between the leg openings. The absorbent assembly may be permanently attached to the fabric. The fabric of the undergarment may be constructed of any suitable fabric, including naturally derived fibers and mixtures thereof selected from the group consisting of cotton, wool, silk, cellulose, regenerated cellulose, rayon, viscose, modal, lyocell, tencel, bamboo, hemp, flax, ramie, coir or banana. Alternatively, the fabric may be constructed of synthetic fibers or mixtures thereof selected from the group consisting of polyamide, acrylic, polyester. Further, the fabric may be constructed of a blend or a mixture of naturally derived and/or synthetic fibers. The fibers may be recycled fibers. The fabric may comprise a stretchable fabric, e.g. elastane, so that the absorbent undergarment can provide a firm fit while at the same time adapting to the wearer's movements thus preventing any leakage from migrating through the leg openings and keeping the absorbent assembly in place. The fabric is preferably breathable to allow vapor to escape from the wearer's skin and from the absorbent structure.

The layers of the absorbent assembly and the undergarment fabric may be bonded by conventional stitching, gluing techniques or via a bonding film such as tape.

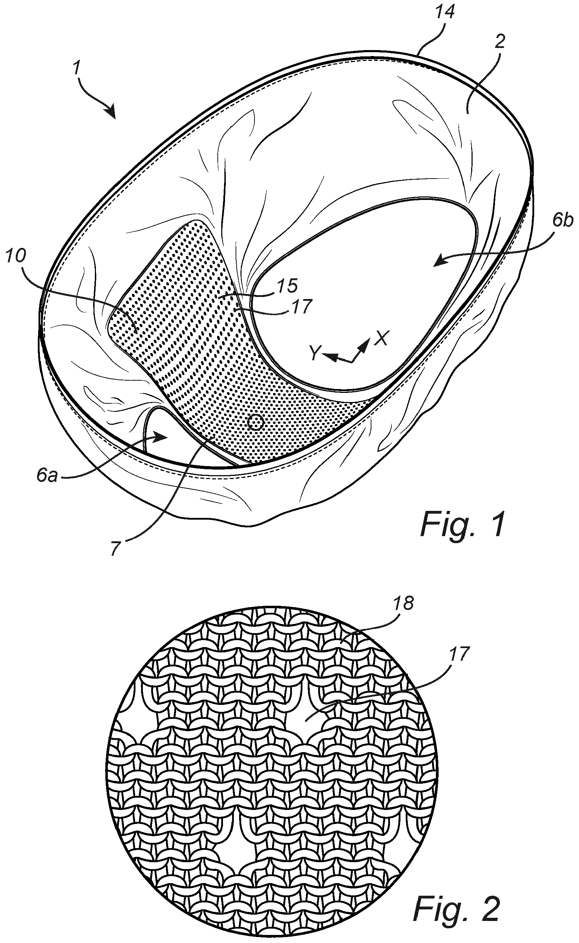

Fig. 1 discloses a washable and reusable absorbent undergarment or underwear 1 in the form of a panty comprising a fabric 2 defining a waist opening, two leg openings 6a, 6b and a crotch region 7 between the leg openings 6a, 6b. The absorbent undergarment 1 further comprises an absorbent assembly 10 covering at least part of the crotch region 7 and may be permanently attached to the fabric layer 2. The absorbent assembly 10 comprises a knitted skin-facing top layer 15. The top layer 15 has openings 17 therein, each opening span 2-10 stitches and there are 5-60 openings/cm². The top layer may be a single layer jacquard stitched material and may have a basis weight of 80-200 gsm. The openings 17 are arranged in transversal rows.

One embodiment for feminine use may have a top layer 15 of a single layer jacquard knit having elongated openings 17 therein that span two stitches, i.e. 2 tuck stitches, and have 20 openings/cm² and each stitch having a largest dimension of about 1.1 mm and a smallest dimension of about 0.8 mm. Such a solution has been shown to be useful for heavy period flows. Another embodiment may have single knit top layer 15 having openings 17 therein that span three stitches and have 50 openings/cm² and each opening having a largest dimension of about 1.5 mm and a smallest dimension of about 0.5 mm. Such a solution has been shown to be useful for incontinence use where large volumes of urine needs to be taken care of.

Figure 2 discloses a close-up of the knitted top layer 15 with the threads 18 and openings 17. The openings are arranged in rows, wherein the openings 17 in adjacent transversal rows are offset in relation to each other. Each opening is of 2 tuck stitches.

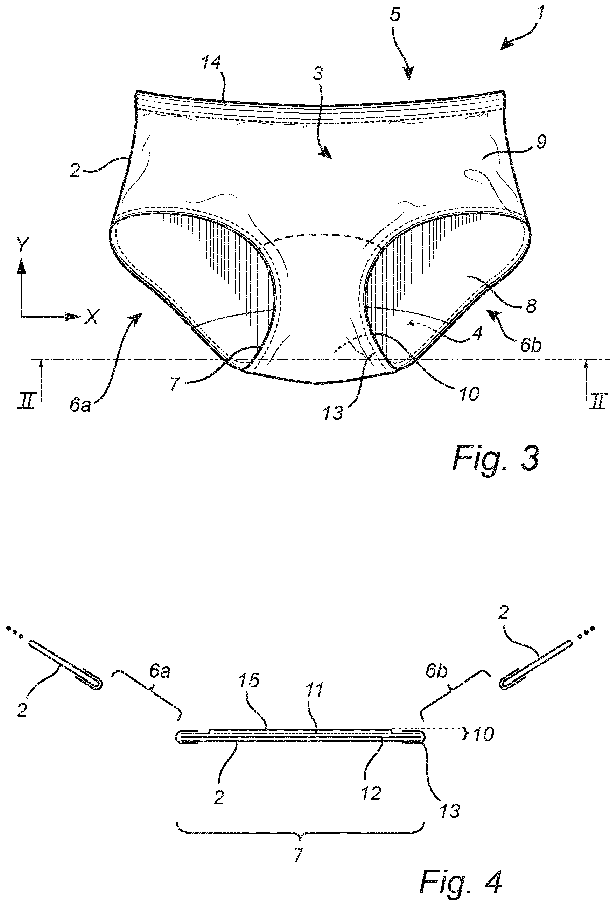

Figure 3 is a front view of an absorbent undergarment 1 comprising a fabric 2 having a front side 3, a back side 4 and a waist opening 5. The fabric 2 has two leg openings 6a and 6b, and a crotch region 7 in between the leg openings 6a and 6b. The fabric 2 further includes an interior surface 8, i.e. a surface of the fabric layer 2 that wholly or partially contacts the body of the wearer, and an exterior surface 9, i.e. a surface of the fabric layer 2 facing away from the wearer. The absorbent undergarment 1 further comprises an absorbent assembly 10 covering at least part of said crotch region 7. In figure 3, a leg opening seam 13 lines the leg openings 6a and 6b. The leg opening seam 13 may be provided by conventional stitching or gluing techniques. The leg opening seam 13 may comprise an elastic member such as elastic bonding film of polyurethane. Further, the leg opening seam 13 may serve to bond the absorbent assembly 10, such as the top layer 15, the absorbent layer(s) 11, the outer liquid impermeable barrier layer 12 and the optionally the wicking layer 16 together. The absorbent undergarment 1 may further comprise a waist band 14. The leg opening and the absorbent assembly may be sealed with a tape (not shown) to avoid side leakage at the leg openings.

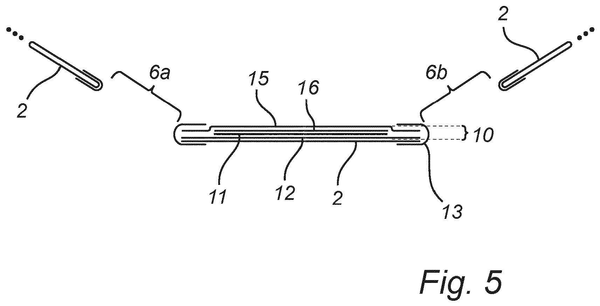

Figure 4 shows a cross-sectional view of the crotch region 7 of an absorbent undergarment 1. The absorbent undergarment 1 comprises the absorbent assembly 10, arranged on the interior surface 8 of the fabric layer 2 and covering a least part of the crotch region 7. The absorbent assembly 10 may comprise one or more absorbent layers 11. In figures 4 and 5, only one absorbent layer 11 is shown to increase clarity of the figures. Further, the absorbent assembly 10 comprises a liquid-impermeable barrier or barrier layer 12 located between the one or more absorbent layers 11 and the fabric layer 2. The liquid-impermeable barrier 12 prevents liquid from leaking through the one or more absorbent layers 11 into the fabric layer 2. The liquid-impermeable barrier 12 may be a coating on the wearer facing side of the fabric layer 2 or on a garment facing side of the absorbent layer 11. The absorbent assembly 10 further comprises a top layer 15 facing the skin of the user. The different layers of the absorbent assembly 10, but additionally also the fabric layer 2, are in Figure 4 bonded together via the leg opening seam 13.

Figure 5 shows an absorbent undergarment 1 wherein the absorbent assembly further comprises a wicking layer 16 underneath the skin-facing top layer 15 and in contact with the absorbent layer 11. A liquid-impermeable barrier 12 is located between the absorbent layer 11 and the undergarment fabric 2.

The disclosure may be varied within the scope of the appended claims. For example, the materials and dimensions used for the different layers forming the absorbent insert may be varied, as indicated above.

### Examples

### Rewet

An artificial menstrual fluid (AMF) according to the French standard AFNOR Q34-018 is prepared. The absorbent assembly is arranged to lie flat on a tabletop in a laboratory conditioned to 23°C and 50% relative humidity. The centre point of the assembly is identified (the point where the longitudinal centre line crosses the transverse centre line). AMF is introduced rapidly via a tube (internal diameter about 3 mm) connected to an automatic dispenser. The orifice of the tube is positioned perpendicular to the centre point with about 5 mm distance to the sample surface. The sample is subjected to three 5.0 ml doses of AMF (i.e.15.0 ml in total), with a 15 minute waiting time between the respective doses. Inlet time is defined as the time from liquid addition till the time when there is no more free fluid on the surface of the absorbent assembly. Rewet is measured 15 minutes after the third (last) dose has been absorbed. A stack of five pre-weighed dry filter papers (90 x 120 mm, 440 g/m² per sheet, Quality 167 from Munktell Ahlstrom or equivalent filter papers) is centred on top of the sample. A 5.5 kg weight with bottom dimension 90 x 120 mm (exerting a pressure of 5 kPa) is gently lowered on top of the stack. After 15 seconds the weight is removed, the filter papers are reweighed, and AMF rewet is determined from the weight difference between the wet and the dry filter papers. Mean values are reported from measurements of ten representative samples.

Garments A and B were tested, see Table 1 below. The garments have the same "base construction" which consists of a wicking layer and two absorption layers. Garment B however has an additional layer with openings according to the present disclosure on top of the wicking layer. The additional layer is a single knit jacquard knitted layer having 22 wales/cm and 30 courses/cm. Each opening is of two tuck stitches and there are four wales between the openings longitudinally and 7 courses between the openings transversally. The openings in adjacent rows are offset in relation to each other. The hole size is 1x0.5 mm and there are about 20 holes/cm². The inlet time, i.e. the time it takes to absorb the fluid as described above was reduced by 30-50% for garment B having the additional layer with openings according to the present disclosure. The layer keeps the fluid inside the product. The rewet property is improved by more than 20%.

The main benefits with a quick inlet time are to prevent leakage and to improve the ability to handle larger volumes of fluid.

**Table 1. Tested samples**

| Material | Fiber composition | Basis weight (gsm) | A Number of layers | B Number of layers |
|---|---|---|---|---|
| Top layer | Polyester, and Elastane | 140 | - | 1 |
| Wicking layer | Polyester | 220 | 1 | 1 |
| Absorption layer | Polyester and Polyamide | 320 | 2 | 2 |

**Table 2. Result**

| Test volume 3x5ml | Reduction of inlet time and rewet (%) of garment B in relation to garment A |
|---|---|
| Inlet 1 | -50 |
| Inlet 2 | -30 |
| Inlet 3 | -38 |
| Rewet | -24 |

## Claims

1. A washable and reusable absorbent undergarment (1) comprising an absorbent assembly (10), the absorbent undergarment (1) having an extension in the longitudinal direction (y) and in the transversal direction (x), the absorbent assembly (10) comprising a wearer facing top layer (15) of a knitted material, a moisture barrier (12) and at least one absorbent layer (11) located between the top layer (15) and the moisture barrier (12), **characterized in that** the top layer (15) has openings (17) therein, and wherein each opening (17) span 2-10 stitches and there are 5-60 openings/cm², wherein the openings (17) each has a dimension within the range of 0.3-2.0 mm.

2. Undergarment according to claim 1, wherein the top layer (15) has 15-25 openings/cm².

3. Undergarment according to claim 1 or 2, wherein the openings (17) span 2-6 stitches.

4. Undergarment according to any one of claims 1-3, wherein the openings (17) are arranged in transversal (x) rows.

5. Undergarment according to any one of claims 1-4, wherein the openings (17) in adjacent rows are offset in relation to each other.

6. Undergarment according to any one of claims 1-5 wherein the openings (17) are elongated and each has a ratio of length to width of >1.

7. Undergarment according to any one of claims 1-6, wherein the openings (17) are elongated and each has a ratio of length to width of >1.5.

8. Undergarment according to any one of claims 1-7, wherein the openings (17) have a bird's eye shape.

9. Undergarment according to any one of claims 1-8, wherein the top layer (15) is of a Jacquard technique.

10. Undergarment according to any one of claims 1-9, wherein the top layer (15) is a single knit fabric layer.

11. Undergarment according to any one of claims 1-10, wherein the openings (17) are tuck stitches.

12. Undergarment according to any one of claims 1-11, wherein the top layer (15) has a basis weight of 80-200 gsm.

13. Undergarment according to any one of claims 1-12, wherein the top layer is not a spacer fabric.

14. Undergarment according to any one of claims 1-13, wherein the openings (17) are through holes.

15. A washable and reusable absorbent assembly (10) having an extension in the longitudinal direction (y) and in the transversal direction (x), comprising a wearer facing top layer (15) of a woven or knitted material, a moisture barrier (12) and at least one absorbent layer (11) located between the top layer (15) and the moisture barrier (12), **characterized in that** the top layer (15) has openings (17) therein and wherein each opening (17) span 2-10 stitches and there are 5-60 openings/cm², wherein the openings (17) each has a dimension within the range of 0.3-2.0 mm.

## Patentansprüche

1. Waschbare und wiederverwendbare absorbierende Unterwäsche (1) mit einer absorbierenden Anordnung (10), wobei sich die absorbierende Unterwäsche (1) in Längsrichtung (y) und in Querrichtung (x) erstreckt, wobei die absorbierende Anordnung (10) eine dem Träger/der Trägerin zugewandte obere Lage (15) aus einem gestrickten Material, eine Feuchtigkeitsbarriere (12) und mindestens eine absorbierende Lage (11), die sich zwischen der oberen Lage (15) und der Feuchtigkeitsbarriere (12) befindet, aufweist, **dadurch gekennzeichnet, dass** die obere Lage (15) Öffnungen (17) darin aufweist, wobei jede Öffnung (17) 2-10 Maschen überspannt und es 5-60 Öffnungen/cm² gibt, wobei die Öffnungen (17) jeweils eine Abmessung im Bereich von 0,3-2,0 mm aufweisen.

2. Unterwäsche nach Anspruch 1, wobei die obere Lage (15) 15-25 Öffnungen/cm² aufweist.

3. Unterwäsche nach Anspruch 1 oder 2, wobei die Öffnungen (17) 2-6 Maschen überspannen.

4. Unterwäsche nach einem der Ansprüche 1-3, wobei die Öffnungen (17) in Quer- (x) Reihen angeordnet sind.

5. Unterwäsche nach einem der Ansprüche 1-4, wobei die Öffnungen (17) in benachbarten Reihen zueinander versetzt sind.

6. Unterwäsche nach einem der Ansprüche 1-5, wobei die Öffnungen (17) länglich sind und jeweils ein Verhältnis von Länge zu Breite von >1 aufweisen.

7. Unterwäsche nach einem der Ansprüche 1-6, wobei die Öffnungen (17) länglich sind und jeweils ein Verhältnis von Länge zu Breite von >1,5 aufweisen.

8. Unterwäsche nach einem der Ansprüche 1-7, wobei die Öffnungen (17) eine Vogelaugenform aufweisen.

9. Unterwäsche nach einem der Ansprüche 1-8, wobei die obere Lage (15) mittels Jacquardtechnik hergestellt ist.

10. Unterwäsche nach einem der Ansprüche 1-9, wobei die obere Lage (15) eine einfache Gestricklage ist.

11. Unterwäsche nach einem der Ansprüche 1-10, wobei die Öffnungen (17) Fangmaschen sind.

12. Unterwäsche nach einem der Ansprüche 1-11, wobei die obere Lage (15) ein Flächengewicht von 80-200 gsm hat.

13. Unterwäsche nach einem der Ansprüche 1-12, wobei die obere Lage kein Abstandsgewirk ist.

14. Unterwäsche nach einem der Ansprüche 1-13, wobei die Öffnungen (17) Durchgangslöcher sind.

15. Waschbare und wiederverwendbare absorbierende Anordnung (10), die sich in Längsrichtung (y) und in Querrichtung (x) erstreckt, die eine dem Träger/der Trägerin zugewandte obere Lage (15) aus einem gewebten oder gestrickten Material, eine Feuchtigkeitsbarriere (12) und mindestens eine absorbierende Lage (11), die sich zwischen der oberen Lage (15) und der Feuchtigkeitsbarriere (12) befindet, aufweist, **dadurch gekennzeichnet, dass** die obere Lage (15) Öffnungen (17) darin aufweist, wobei jede Öffnung (17) 2-10 Maschen überspannt und es 5-60 Öffnungen/cm² gibt, wobei die Öffnungen (17) jeweils eine Abmessung im Bereich von 0,3-2,0 mm aufweisen.

## Revendications

1. Un sous-vêtement absorbant lavable et réutilisable (1) comprenant un ensemble absorbant (10), le sous-vêtement absorbant (1) ayant une extension dans la direction longitudinale (y) et dans la direction transversale (x), l'ensemble absorbant (10) comprenant une couche supérieure (15) faisant face au porteur d'un matériau tricoté, un obstacle à l'humidité (12) et au moins une couche absorbante (11) située entre la couche supérieure (15) et l'obstacle à l'humidité (12), **caractérisé par le fait que** la couche supérieure (15) comporte des ouvertures (17) dans celle-ci, et où chaque ouverture (17) couvre 2 à 10 mailles et il y a 5 à 60 ouvertures/cm², où les ouvertures (17) ont chacune une dimension comprise entre 0,3 et 2,0 mm.

2. Sous-vêtement selon la revendication 1, dans lequel la couche supérieure (15) comporte 15 à 25 ouvertures/cm².

3. Sous-vêtement selon la revendication 1 ou 2, dans lequel les ouvertures (17) couvrent 2 à 6 mailles.

4. Sous-vêtement selon l'une des revendications 1 à 3, dans lequel les ouvertures (17) sont disposées en rangées transversales (x).

5. Sous-vêtement selon l'une des revendications 1 à 4, dans lequel les ouvertures (17) dans les rangées adjacentes sont décalées les unes par rapport aux autres.

6. Sous-vêtement selon l'une des revendications 1 à 5, dans lequel les ouvertures (17) sont allongées et chacune a un rapport longueur sur largeur de >1.

7. Sous-vêtement selon l'une des revendications 1 à 6, dans lequel les ouvertures (17) sont allongées et chacune a un rapport longueur sur largeur de >1,5.

8. Sous-vêtement selon l'une des revendications 1 à 7, dans lequel les ouvertures (17) ont une forme d'œil d'oiseau.

9. Sous-vêtement selon l'une des revendications 1 à 8, dans lequel la couche supérieure (15) est d'une technique Jacquard.

10. Sous-vêtement selon l'une des revendications 1 à 9, dans lequel la couche supérieure (15) est une seule couche de tissu tricoté.

11. Sous-vêtement selon l'une des revendications 1 à 10, dans lequel les ouvertures (17) sont des mailles replie.

12. Sous-vêtement selon l'une des revendications 1 à 11, dans lequel la couche supérieure (15) a un poids de base de 80 à 200 gsm.

13. Sous-vêtement selon l'une des revendications 1 à 12, dans lequel la couche supérieure n'est pas un tissu entretoiseur.

14. Sous-vêtement selon l'une des revendications 1 à 13, dans lequel les ouvertures (17) sont des trous traversants.

15. Un ensemble absorbant lavable et réutilisable (10) ayant une extension dans la direction longitudinale (y) et dans la direction transversale (x), comprenant une couche supérieure (15) faisant face au porteur d'un tissu tissé ou tricoté, un obstacle à l'humidité (12) et au moins une couche absorbante (11) située entre la couche supérieure (15) et l'obstacle à l'humidité (12), **caractérisé par le fait que** la couche supérieure (15) comporte des ouvertures (17) dans celle-ci et où chaque ouverture (17) couvre 2 à 10 mailles et il y a 5 à 60 ouvertures/cm², où les ouvertures (17) ont chacune une dimension comprise entre 0,3 et 2,0 mm.
